# EUROPÄISCHE PATENTANMELDUNG

(11) **EP 3 305 331 A1**
(43) Veröffentlichungstag der Anmeldung: **11.04.2018**
(21) Anmeldenummer: 17190341.2
(22) Anmeldetag: 11.09.2017
(51) Int. Cl.: A61L 2/28

(54) **VERFAHREN ZUM ÜBERPRÜFEN ZUMINDEST EINES DESINFEKTIONSMITTELS**

(30) Priorität: 13.09.2016 DE 102016117196
(71) Anmelder: Brandes Innovation Inh. Ronald Brandes, 26419 Schortens (DE)
(72) Erfinder: Willms, Joachim, 26133 Oldenburg (DE); Brandes, Ronald, 26419 Schortens (DE)
(74) Vertreter: Jabbusch, Matthias

(57) **Zusammenfassung**

Bei einem Verfahren zum Überprüfen zumindest eines Desinfektionsmittels auf seine Desinfektions-Funktion ist vorgesehen, dass zumindest eine Probe des Desinfektionsmittels mit einem für dieses Desinfektionsmittel geeigneten Inhibitor versetzt wird und in der Probe anschließend mit einem Sensor die Reaktion des Inhibitors gemessen wird.

## Beschreibung

Die Erfindung betrifft ein Verfahren zum Überprüfen zumindest eines Desinfektionsmittels auf seine Desinfektions-Funktion.

Desinfektionsmittel werden zum Abtöten von Keimen in vielfältigen Umgebungen angewandt. Ein regelmäßiger Einsatz ist in Krankenhäusern, Kinderheimen oder auch Altenheimen gegeben, in denen vermehrt Krankheitserreger auftreten können. Desinfektionsmittel werden beispielsweise in Behältern vorgehalten, aus denen heraus Desinfektionsmittel zum Besprühen der Hände, zum Besprühen von Tüchern oder ähnlich entnommen werden können.

Problematisch ist, dass es in derartigen Krankenhäusern und Heimen häufig zu Drucksituationen hinsichtlich hoher Patienten- oder Heimbewohnerzahlen, geringem Personal, einer großen Anzahl von Praktikanten oder Zivildienstleistenden kommt. Dies alles kann dazu führen, dass ein Desinfektionsmittel in einem konkreten Behälter wochen- oder auch monatelang enthalten ist, ohne dass auf seine Ablaufzeit geachtet wird. Ein derartiges Desinfektionsmittel kann seine Desinfektions-Funktion verlieren, vielmehr kann es sogar mit Keimen belastet sein. Dann würde ein Desinfektionsmittel eine Keimquelle ausbilden.

Der Erfindung liegt die Aufgabe zugrunde, ein Verfahren der eingangs genannten Gattung aufzuzeigen, mit dem ein Desinfektionsmittel zuverlässig auf seine Desinfektions-Funktion überprüfbar ist.

Diese Aufgabe ist erfindungsgemäß dadurch gelöst, dass zumindest eine Probe des Desinfektionsmittels mit einem für dieses Desinfektionsmittel geeigneten Inhibitor versetzt wird und in der Probe anschließend mit einem Sensor die Reaktion des Inhibitors gemessen wird.

Das erfindungsgemäße Verfahren schlägt eine aktive Überprüfung von Desinfektionsmitteln während ihrer Verwendung in zum Beispiel Krankenhäusern vor. Nach dem Verfahren wird dem Desinfektionsmittel zumindest eine Probe entnommen, alternativ kann direkt im konkret vorhandenen Desinfektionsmittelvorrat das Verfahren durchgeführt werden. Die gewonnene Probe beziehungsweise das Desinfektionsmittel wird mit einem für dieses Desinfektionsmittel geeigneten Inhibitor versetzt. Ein Inhibitor ist ein Gegenmittel zu einem im Desinfektionsmittel enthaltenen Wirkstoff, mit dem eine ausreichende Konzentration dieses Wirkstoffes im Desinfektionsmittel überprüfbar ist. Dabei wird für ein bestimmtes Desinfektionsmittel ein Inhibitor eingesetzt, der auf die im Desinfektionsmittel enthaltenen und die Desinfektions-Wirkung begründenden Wirkstoffe reagiert. Mit dem Inhibitor ist somit die ausreichende Konzentration der für die Desinfektions-Funktion verantwortlichen Substanzen überprüfbar.

Das Überprüfen erfolgt dann verfahrensgemäß durch einen Sensor, der in automatisierter Weise das Messergebnis feststellt und über geeignete Schnittstellen beziehungsweise Übertragungswege an eine Auswertung liefert. Der Sensor misst die Reaktion des Inhibitors nach dessen Kontakt mit dem Desinfektionsmittel. Hat das Desinfektionsmittel noch eine hohe desinfizierende Wirksamkeit, kommt es zu einer stärkeren Reaktion des Inhibitors, die messbar ist. Ist dagegen die Desinfektionseigenschaft des Desinfektionsmittels durch eine höhere Konzentration von Bakterien im Desinfektionsmittel gebunden, fällt die Reaktion am Inhibitor geringer aus, was wiederrum mit dem Sensor festgestellt wird.

Durch den Kontakt des Desinfektionsmittels mit einem Inhibitor wird beispielsweise die Leitfähigkeit des Desinfektionsmittels verändert. Diese Änderung kann mit einem Leitfähigkeitssensor festgestellt werden. Alternativ kann der Sensor auch als Lichtsensor ausgebildet sein, wenn nämlich die Reaktion von Desinfektionsmittel und Inhibitor zu einer Veränderung der Trübung führen.

Weiter alternativ kann der Sensor als pH-Wert-Messer oder als Dichtesensor ausgebildet sein.

Als Inhibitor kann beispielweise ein TSHC genannter oder TSLHTh genannter Inhibitor des Herstellers Bode verwendet werden. Dieser enthält Bestandteile wie Tween 80, Saponin, Histidin und Cystein. Ein derartiger Inhibitor kann für Desinfektionsmittel mit den Namen Manusept basic oder soft oder Sterillium mit den zusätzlichen Namen Comfort Gel, Gel, Gel pure, med, Rub Fragrance Free und Virugard eingesetzt werden.

Auch Produkte der Firma Schülke & Mayr GmbH, Norderstedt, können als Inhibitoren verwendet werden. So zum Beispiel Produkte mit den Namen mikrocount® duo, mikrocount® TPC, mikrocount® TPC/E. Diese Produkte sind Medien zur Ermittlung von Bakterienzahlen, sie können nach dem erfindungsgemäßen Verfahren aber auch als Inhibitoren für die Wirksamkeit eines Desinfektionsmittels eingesetzt werden.

## Patentansprüche

1. Verfahren zum Überprüfen zumindest eines Desinfektionsmittels auf seine Desinfektions-Funktion,
**dadurch gekennzeichnet,**
**dass** zumindest eine Probe des Desinfektionsmittels mit einem für dieses Desinfektionsmittel geeigneten Inhibitor versetzt wird und in der Probe anschließend mit einem Sensor die Reaktion des Inhibitors gemessen wird.

2. Verfahren nach Anspruch 1, **dadurch gekennzeichnet, dass** als Sensor ein Leitfähigkeitssensor eingesetzt wird.

3. Verfahren nach Anspruch 1, **dadurch gekennzeichnet, dass** als Sensor ein Lichtsensor eingesetzt wird.

4. Verfahren nach Anspruch 1, **dadurch gekennzeichnet, dass** als Sensor ein pH-Wert-Messer eingesetzt wird.

5. Verfahren nach Anspruch 1, **dadurch gekennzeichnet, dass** als Sensor ein Dichtesensor eingesetzt wird.

6. Verfahren nach einem der vorhergehenden Ansprüche, **dadurch gekennzeichnet, dass** als Inhibitor Natriumthiosulfat, Lecithin, L-Histidin, Tween 80, Saponin und/oder Ethersulfat verwendet werden.
